# EUROPEAN PATENT APPLICATION

(11) **EP 4 541 275 A1**
(43) Date of publication of application: **23.04.2025**
(21) Application number: 23821107.2
(22) Date of filing: 07.09.2023
(51) Int. Cl.: A61B 5/145

(54) **ANALYTE SENSOR, AND PREPARATION METHOD THEREFOR AND APPLICATION METHOD THEREOF**

(71) Applicant: Shanghai United Imaging Microelectronics Technology Co., Ltd., Jiading District Shanghai 201800 (CN)
(72) Inventor: WANG, Yunli, Shanghai 201800 (CN)
(74) Representative: Viering, Jentschura & Partner mbB Patent- und Rechtsanwälte
(86) International application number: PCT/CN2023/117396
(87) International publication number: WO 2025/050335

(57) **Abstract**

An analyte sensor (1), a preparation method and an application thereof are provided. The analyte sensor (1) includes a sensing probe (10), an auxiliary needle (20) and a drug formulation (30). The auxiliary needle (20) includes a needle tip (21), a side wall (22) and a bottom wall (23). The needle tip (21) is connected to the bottom wall (23). An accommodating groove (24) is defined by the side wall (22) and the bottom wall (23) connected to each other. The accommodating groove (24) is configured to accommodate the sensing probe (10). The drug formulation (30) is disposed on the auxiliary needle (20) and/or on the sensing probe (10).

## Description

### TECHNICAL FIELD

The present invention relates to the field of sensors, and in particular, to an analyte sensor, a preparation method and an application thereof.

### BACKGROUND

Analyte sensor is a type of sensor for subcutaneous analyte measurement or transdermal detection. By implanting a sensing probe into a target site under the skin, the analyte sensor is configured for continuous, in-field monitoring of a target analyte and feedback of monitoring data to an external source.

In the related art, incisions caused by the analyte sensor during implantation may lead to acute inflammatory reactions and bleeding, making the analyte sensor less accurate for a period of time after implantation. In addition, as the sensing probe of the analyte sensor can lead to foreign body reaction (FBR) such as inflammatory reaction, foreign body giant cell formation, fibrosis, etc., after implantation in the human body, it ultimately forms a collagenous wrapping around the implanted probe, which prevents transmission and exchange of substances between the probe and surrounding tissues, i.e., prevents transmission of the target analyte from reaching the sensing probe, affecting the sensitivity, accuracy, stability, and other functions of the long-term analyte sensor. In addition, the sensors, adhesive tapes, other components, and the glue that may be used can produce skin irritation and lead to allergies.

Many researches focused on reducing a thickness of the fibrotic layer around an implantable medical device by providing a surface coating that reduces protein contamination and cell attachment; or, achieving anti-inflammatory and anti-fibrotic properties by coating the outside of a functional area of a working electrode of the analyte sensor probe with a membrane layer containing a drug. However, the problem of targeted delivery of drug formulations to different target sites is not addressed in the related art.

Accordingly, there is an urgent need to provide an analyte sensor that can deliver drug formulations to different target sites.

### SUMMARY

According to various embodiments of the present application, an analyte sensor, a preparation method and an application thereof are provided.

In a first aspect, the present invention provides an analyte sensor. The analyte sensor includes a sensing probe, an auxiliary needle and a drug formulation. The auxiliary needle includes a needle tip, a side wall and a bottom wall. The needle tip is connected to the bottom wall. An accommodating groove is defined by the side wall and the bottom wall connected to each other. The accommodating groove is configured to accommodate the sensing probe. The drug formulation is disposed on the auxiliary needle and/or on the sensing probe.

In some embodiments, the side wall of the auxiliary needle includes a first portion, the first portion of the side wall is located at an end of the side wall proximate to the needle tip, and a height of the first portion perpendicular to the bottom wall decreases in a direction proximate to the needle tip.

In some embodiments, a first protrusion is further provided on an outer surface of the side wall of the auxiliary needle.

In some embodiments, the sensing probe includes a probe substrate and an electrode, the probe substrate includes an electrode region and a blank region, the electrode is disposed on the electrode region of the probe substrate.

In some embodiments, the blank region includes a first blank region, a second blank region, a third blank region, a fourth blank region and a fifth blank region, the first blank region is located at one end of the probe substrate adjacent to the needle tip of the auxiliary needle, the second blank region is located on both sides of the electrode region of the probe substrate, the third blank region is located on both sides of the probe substrate, the fourth blank region is located at one end of the probe substrate away from the needle tip of the auxiliary needle, and the fifth blank region is located on an opposite side of the probe substrate relative to a side of the probe substrate having the electrode.

In some embodiments, the sensing probe includes a probe substrate, the probe substrate further includes a second protrusion, and the second protrusion is disposed between the probe substrate and the side wall of the auxiliary needle.

In some embodiments, the drug formulation includes a functional component.

In some embodiments, the functional component includes at least one of an anti-inflammatory component, an anti-coagulant component, an anti-fibrotic component, a pro-angiogenic component, or an anti-allergic component.

In some embodiments, the anti-inflammatory component includes at least one of curcumin, dexamethasone, prednisone, methyl-prednisone, betamethasone, beclomethasone propionate, prednisolone, hydrocortisone, or methylprednisolone; the anti-coagulant component includes at least one of sodium heparin, calcium heparin, enoxaparin sodium, dalteparin sodium, nalteparin calcium, argatroban, bivalirudin, warfarin, dabigatran etexilate, rivaroxaban, apixaban, dipyridamole, aspirin, clopidogrel hydrogensulphate, urokinase, or antithrombin; the anti-fibrotic component includes at least one of a macrophage colony-stimulating factor-1 receptor inhibitor, colchicine, interferon, an endothelin receptor antagonist, pirfenidone, nintedanib, compound embryonic bovine liver extract, asiaticoside, D-penicillamine, retinoic acid, 5-fluorouracil, or relaxin peptide analogs B7-33; the pro-angiogenic component includes vascular endothelial growth factor, platelet-derived growth factor, or any combination thereof; and the anti-allergic component includes at least one of mometasone furoate, desonide, triamcinolone acetonide, fluticasone propionate, or halometasone.

In some embodiments, the drug formulation further includes an excipient, a pro-osmotic agent, or any combination thereof.

In some embodiments, the excipient includes at least one of collagen, bovine serum albumin, gelatin, chitosan, hyaluronic acid, chondroitin sulfate, sodium alginate, polyvinyl alcohol, polyethylene glycol, polyvinylpyrrolidone, poly(lactic acid-hydroxyacetic acid) copolymer, triglyceride, dextrin, sodium hydroxymethylcellulose, petroleum jelly, propylene glycol, glycerol, stearic acid, mono glyceryl stearate, lanolin, beeswax, paraffin, liquid paraffin, triethanolamine, Span-80, or emulsifier OP-10.

In some embodiments, the pro-osmotic agent includes at least one of lecithin, ethanol, propylene glycol, ethyl acetate, dimethyl sulfoxide, lauric nitrone, oleic acid, lauryl alcohol, urea, salicylic acid, dimethyl amino acid ester, or limonene.

In some embodiments, a form of the drug formulation includes a coating, a gel, an emulsion, a paste, a microcapsule, or any combination thereof.

In some embodiments, the drug formulation is disposed at an end of the accommodating groove of the auxiliary needle proximate to the needle tip.

In some embodiments, the drug formulation includes a functional component, and the functional component includes an anti-inflammatory component, an anti-coagulant component, and an anti-fibrosis component, or any combination thereof.

In some embodiments, the drug formulation is disposed on an inner surface of the side wall of the auxiliary needle; and/or, the drug formulation is disposed on an inner surface of the bottom wall of the auxiliary needle.

In some embodiments, the drug formulation further includes an excipient, a pro-osmotic agent, or any combination thereof.

In some embodiments, the drug formulation is disposed on an outer surface of the side wall of the auxiliary needle.

In some embodiments, the drug formulation includes a functional component, and the functional component includes an anti-inflammatory component, an anti-allergic component, or any combination thereof.

In some embodiments, the drug formulation is disposed at a position of the first protrusion away from the needle tip.

In some embodiments, the drug formulation includes a functional component, and the functional component includes an anti-inflammatory component, an anti-coagulant component, an anti-fibrotic component, a pro-angiogenic component, or any combination thereof.

In some embodiments, the electrode includes a working electrode, a reference electrode, and a counter electrode, the drug formulation is disposed on a surface of the working electrode, a surface of the reference electrode, a surface of the counter electrode, the first blank region, the second blank region, the third blank region, the fourth blank region, the fifth blank region, or any combination thereof.

In some embodiments, the drug formulation is disposed on a surface of the reference electrode, a surface of the counter electrode, the first blank region, the second blank region, the third blank region, the fourth blank region, the fifth blank region, or any combination thereof.

In some embodiments, the drug formulation is disposed on an outer surface of the side wall of the auxiliary needle and the fifth blank region.

In some embodiments, the drug formulation includes a functional component, and the functional component includes an anti-inflammatory component, an anti-coagulant component, or any combination thereof.

In a second aspect, the present invention further provides a method for preparing the analyte sensor. The method includes following steps: preparing the drug formulation; disposing the drug formulation on the auxiliary needle and/or on the sensing probe; and disposing the sensing probe in the accommodating groove of the auxiliary needle.

In some embodiments, the drug formulation is disposed on the auxiliary needle by a method of in-hole spot coating, surface spot coating, dip coating, spin coating, spray coating, or combination thereof.

In some embodiments, the drug formulation is disposed on the sensing probe by a method of in-hole spot coating, surface spot coating, dip coating, spin coating, spray coating, or combination thereof.

In a third aspect, the present invention further provides a method of applying the analyte sensor. The method includes following steps: percutaneously piercing the analyte sensor into a target site; and withdrawing the auxiliary needle and leaving the sensing probe at the target site.

Details of one or more embodiments of this application are presented in the attached drawings and descriptions below. And other features, purposes and advantages of this application will become apparent from the description, drawings and claims.

### BRIEF DESCRIPTION OF THE DRAWINGS

In order to more clearly illustrate the technical solutions in the embodiments of the present invention or in the conventional technology, the accompanying drawings to be used in the description of the embodiments or in the conventional technology will be briefly described below, and it will be obvious that the accompanying drawings in the following description are only the embodiments of the present application, and that other accompanying drawings can be obtained by a person of ordinary skill in the field based on the disclosed accompanying drawings, without any creative labor.
FIG. 1 is a schematic diagram of a portion of an analyte sensor in an embodiment of the present application.
FIG. 2 is a schematic diagram of a portion of an analyte sensor in an embodiment of the present application.
FIG. 3 is a schematic diagram of a portion of an analyte sensor in an embodiment of the present application.
FIG. 4 is a schematic diagram of an auxiliary needle of the analyte sensor of FIG. 3 which is withdrawn in an X direction.
FIG. 5 is a schematic diagram of a portion of an analyte sensor in an embodiment of the present application.
FIG. 6 is a schematic diagram of an auxiliary needle of an analyte sensor which is advanced along in a Y direction in an embodiment of the present application.
FIG. 7 is a schematic diagram of an auxiliary needle of an analyte sensor which is withdrawn in an X direction in an embodiment of the present application.
FIG. 8 is a schematic diagram of an analyte sensor which is advanced in a Y direction in an embodiment of the present application.
FIG. 9 is a front view of a sensing probe of an analyte sensor in an embodiment of the present application.
FIG. 10 is a left view of a sensing probe of an analyte sensor in an embodiment of the present application.
FIG. 11 is a front view of a sensing probe of an analyte sensor in an embodiment of the present application.
FIG. 12 is a cross-sectional view of the analyte sensor of FIG. 11 along B-B line.
FIG. 13 is a front view of a sensing probe of an analyte sensor in an embodiment of the present application.
FIG. 14 is a cross-sectional view of the analyte sensor of FIG. 13 along C-C line.
FIG. 15 is a front view of a sensing probe of an analyte sensor in an embodiment of the present application.
FIG. 16 is a cross-sectional view of the analyte sensor of FIG. 13 along D-D line.

Reference signs are as follows: 1 represents an analyte sensor; 10 represents a sensing probe; 11 represents a probe substrate; 111 represents an electrode region; 112 represents a blank region; 1121 represents a first blank region; 1122 represents a second blank region; 1123 represents a third blank region; 1124 represents a fourth blank region; 1125 represents a fifth blank region; 113 represents a second protrusion; 12 represents an electrode; 121 represents a counter electrode; 122 represents a working electrode; 123 represents a reference electrode; 13 represents a wire; 20 represents an auxiliary needle; 21 represents a needle tip; 22 represents a side wall; 221 represents a first portion; 222 represents a second portion; 223 represents a first protrusion; 23 represents a bottom wall; 24 represents an accommodating groove; 30 represents a drug formulation; 31 represents a drug formulation retained at a target site; and A represents a skin.

### DETAILED DESCRIPTION

The technical scheme in the embodiment of this application will be described clearly and completely with the attached drawings. Obviously, the described embodiment is only a part of the embodiment of this application, not the whole embodiment. Based on the embodiments in this application, all other embodiments obtained by ordinary technicians in this field without creative work belong to the protection scope of this application.

It should be noted that when a component is said to be "fixed to" or "disposed on" on another component, it can be directly on the other component or there can be a component in the middle. When a component is considered to be "connected to" another component, it can be directly connected to another component or there may be intervening components at the same time. The terms "vertical", "horizontal", "up", "down", "left", "right", and similar expressions used in the specification of this application are for illustrative purposes only and are not intended to be the sole means of implementation.

In the present application, the terms "first" and "second" are used for descriptive purposes only, and are not to be understood as indicating or implying relative importance or implicitly specifying the number of indicated technical features. Thus, a feature defined with the terms "first" and "second" may include at least one such feature, either explicitly or implicitly. In the description of the present application, "plurality" means at least two, e.g., two, three, etc., unless otherwise expressly and specifically limited.

In the present invention, unless otherwise expressly specified and limited, the first feature "over" or "under" the second feature may be a direct contact between the first feature and the second feature, or an indirect contact between the first feature and the second feature through an intermediate medium. Furthermore, the first feature being "on", "above", and "over" the second feature may be that the first feature is directly over or diagonally over the second feature, or simply that the first feature is horizontally higher than the second feature. The first feature is "below", "under" and "underneath" the second feature may be that the first feature is directly under or diagonally under the second feature, or may simply mean that the first feature is less horizontal than the second feature.

In the present invention, "supradermal" refers to a location where the skin is in contact with the outside world, "subcutaneous" refers to a location where the skin is internally organized; "transdermal" refers to passing through the skin. "Target site" refers to a location where the sensing probe is implanted and/or where the drug formulation is functional.

Unless otherwise defined, all technical and scientific terms used herein have the same meaning as commonly understood by those skilled in the art of the present invention. Terms used in the specification of the present invention are used only for the purpose of describing specific embodiments and are not intended to limit the invention. The term "and/or" as used herein includes any and all combinations of one or more of the relevant listed items.

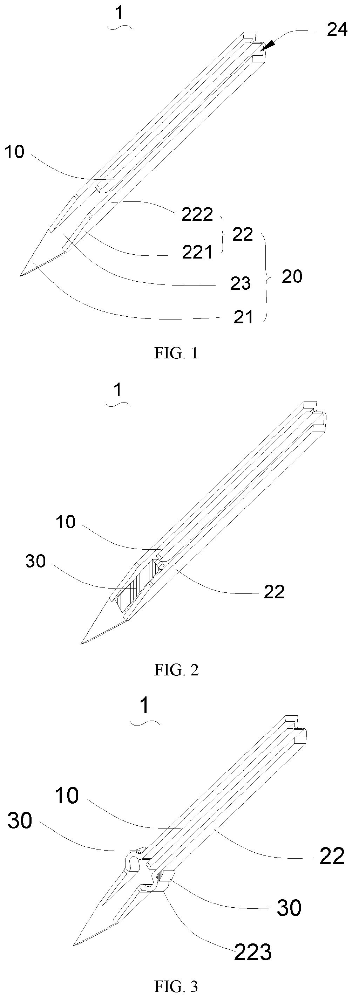

Referring to FIG. 1, the present invention provides an analyte sensor 1 including a sensing probe 10, an auxiliary needle 20, and a drug formulation 30. The auxiliary needle 20 includes a needle tip 21, a side wall 22, and a bottom wall 23. The needle tip 21 is connected to the bottom wall 23. The side wall 22 is connected to the bottom wall 23 to form an accommodating groove 24. The accommodating groove 24 is configured to accommodate the sensing probe 10. The drug formulation 30 is disposed on the auxiliary needle 20 and/or on the sensing probe 10.

For the analyte sensor 1 in the present invention, by providing the auxiliary needle 20, it is beneficial to protect the sensing probe 10 and smoothly deliver the sensing probe 10 percutaneously to a target site. Meanwhile, different drug formulations 30 can also be disposed on the sensing probe 10 and/or the auxiliary needle 20 according to a type, a dosage form, and a function of the drug formulation 30 as required, i.e., different drug formulations 30 can be delivered to different target sites, thereby targeting anti-inflammatory, anti-coagulant, anti-allergic, anti-fibrotic, etc., and thus achieving a purpose of improving accuracy, stability, and sensitivity of the analyte sensor 1, as well as to extend a service life of the analyte sensor 1 and to improve the comfortability of the user.

In some embodiments, the side wall 22 includes a first portion 221. The first portion 221 is located at an end of the side wall 22 proximate to the needle tip 21. A height of the first portion 221 perpendicular to the bottom wall 23 decreases in a direction proximate to the needle tip 21. In this way, it facilitates piercing of the subcutaneous tissue by the auxiliary needle 20. In some embodiments, the first portion 221 may be wedge-shaped or other shapes, as long as the first portion 221 facilitates the piercing of the subcutaneous tissue by the auxiliary needle 20.

In some embodiments, the first portion 221 may extend to the needle tip 21. In this way, the sensing probe 10 can be better protected to help deliver the drug formulation to the target site under the skin.

In some embodiments, the side wall 22 of the auxiliary needle 20 further includes a second portion 222. The second portion 222 is disposed at an end of the side wall 22 away from the needle tip 21. In some embodiments, the second portion 222 may be in a rectangular shape. In some embodiments, the second portion 222 may also be in other shapes, as long as the second portion 222 facilitates delivery of the drug formulation 30 to the target site and may further capable of protecting the sensing probe 10.

In some embodiments, a first protrusion 223 is further provided on an outer surface of the side wall 22 of the auxiliary needle 20, as shown in FIG. 3. By providing the first protrusion 223 on the outer surface of the side wall 22 of the auxiliary needle 20, it is beneficial to assist the skin tissue in retaining the medication formulation 30 underneath the skin during withdrawal of the auxiliary needle 20.

In some embodiments, one first protrusion 223 is provided on an outer surface of each of the two side walls 22 of the auxiliary needle 20.

In some embodiments, the first protrusion 223 is provided between the first portion 221 and the second portion 222. In this way, by providing the first protrusion 223, it is beneficial to retain the drug formulation 30 under the skin during needle withdrawal.

In some embodiments, the needle tip 21 of the auxiliary needle 20 has an angle in a range of 20° - 50°. In some embodiments, the needle tip 21 of the auxiliary needle 20 has an angle in a range of 30° - 45°. A thickness of the auxiliary needle 20 is in a range of 0.05 mm - 0.2 mm. In some embodiments, the thickness of the auxiliary needle 20 is in a range of 0.06 mm - 0.12 mm. In some embodiments, a width of the auxiliary needle 20 is in a range of 0.4 mm - 0.8 mm. In some embodiments, the width of the auxiliary needle 20 is in a range of 0.45 mm - 0.65 mm. In this way, under the premise of ensuring the delivery area and stability when the analyte sensor 1 delivers the drug, it facilitates the auxiliary needle 20 to smoothly pierce into the subcutaneous tissue and cause a smaller wound on the skin, which in turn is beneficial to reduce an impact of acute inflammatory reaction and bleeding on the analyte sensor 1.

In some embodiments, the side wall 22, the bottom wall 23, and the needle tip 21 of the auxiliary needle 20 may be integrally formed. Alternatively, the auxiliary needle 20, the bottom wall 23, and the needle tip 21 may be provided in a separate manner and combined by welding, bonding, and the like.

In some embodiments, the auxiliary needle 20 is made of stainless steel, titanium, tantalum, magnesium, nickel-titanium alloy, cobalt alloy, ceramic, and the like.

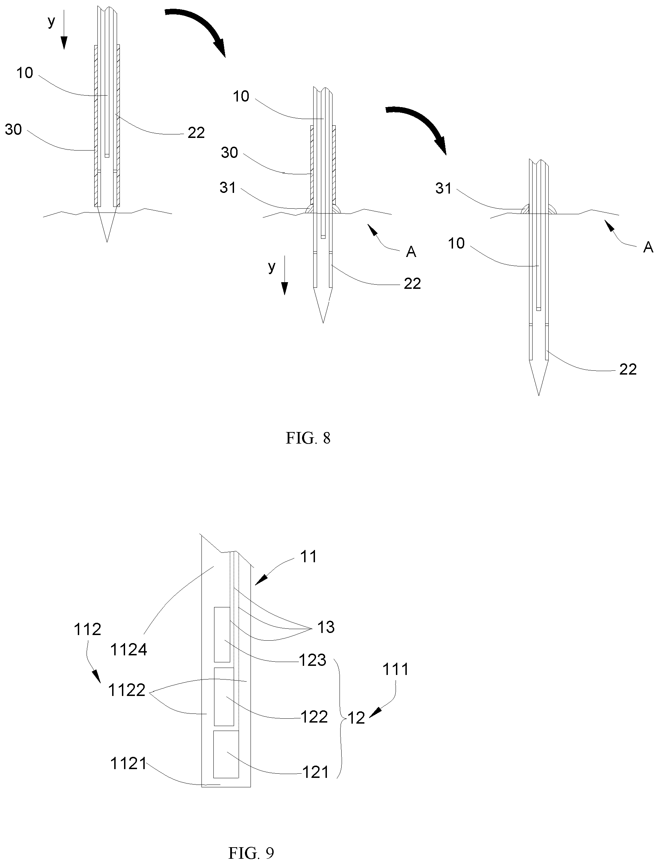

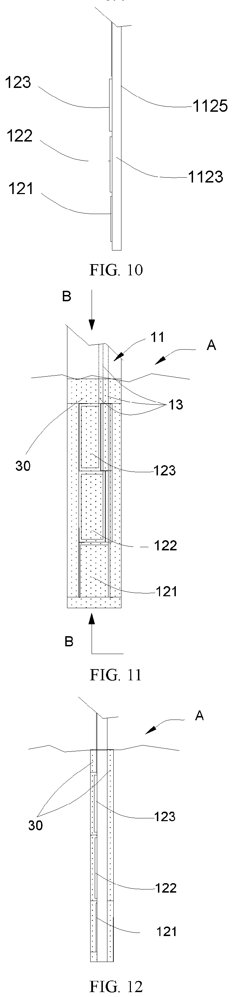

In some embodiments, referring to FIGs. 9 and 10, the sensing probe 10 includes a probe substrate 11, an electrode 12, and a wire 13. The probe substrate 11 includes an electrode region 111 and a blank region 112, and the electrode 12 is provided in the electrode region 111 of the probe substrate 11.

In some embodiments, the blank region 112 includes a first blank region 1121 , a second blank region 1122, a third blank region 1123, a fourth blank region 1124, and a fifth blank region 1125. The first blank region 1121 is located at an end of the probe substrate 11 proximate to the needle tip 21 of the auxiliary needle 20. The second blank region 1122 is located at both sides of the electrode region 111 of the probe substrate 11. The third blank region 1123 is located on both sides of the probe substrate 11. The fourth blank region 1124 is located on an end of the probe substrate 11 away from the needle tip 21 of the auxiliary needle 20. The fifth blank region 1125 is located on an opposite side of the probe substrate 11 relative to a side of the probe substrate 11 on which the electrode 12 is provided.

In some embodiments, the electrode 12 of the sensing probe 10 includes a counter electrode 121, a reference electrode 123, and a working electrode 122.

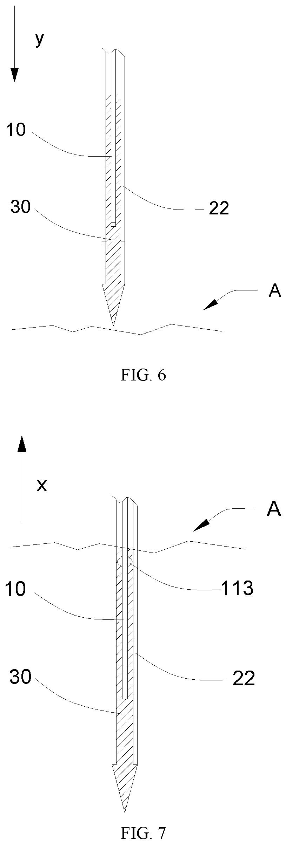

In some embodiments, as shown in FIG. 7, the probe substrate 11 further includes a second protrusion 113. The second protrusion 113 is disposed between the probe substrate 11 and the side wall 22 of the auxiliary needle 20. In some embodiments, two second protrusions 113 are provided on the probe substrate 11, and the two second protrusions 113 are located on each side of the probe substrate 11. By providing the second protrusions 113, it is beneficial to retain the drug formulation 30 at an implantation site of the sensing probe 10 during the withdrawal of the auxiliary needle 20.

In some embodiments, the sensing probe 10 is provided in the accommodating groove by fit and restriction of a structural member. In some embodiments, the fifth blank region 1125 is not in contact with the bottom wall 23 of the auxiliary pin 20. By providing the sensing probe 10 in the above manner, it is easy to set the drug formulation 30 in the accommodating groove and/or the sensing probe 10, and it is also convenient for withdrawal of the auxiliary needle 20.

In some embodiments, the drug formulation 30 includes a functional component.

In some embodiments, the functional component includes an anti-inflammatory component, an anti-coagulant component, an anti-fibrotic component, a pro-angiogenic component, an anti-allergic component, or any combination thereof. In some embodiments, the drug formulation 30 containing different functional components can be disposed at different locations of the analyte sensor 1 according to different needs, so as to better target the effect of the functional components.

In some embodiments, the anti-inflammatory component includes at least one of curcumin, dexamethasone, prednisone, methyl-prednisone, betamethasone, beclomethasone propionate, prednisolone, hydrocortisone, or methylprednisolone.

In some embodiments, the anti-coagulant component includes at least one of heparin sodium, heparin calcium, enoxaparin sodium, dalteparin sodium, naltrexone calcium, argatroban, bivalirudin, warfarin (benzylketone coumarin sodium), dabigatranate, rivaroxaban, apixaban, dipyridamole, aspirin, clopidogrel hydrogencarbyl sulfate, urokinase, or antithrombin.

In some embodiments, the anti-fibrotic component includes at least one of a macrophage colony-stimulating factor-1 receptor inhibitor, colchicine, interferon, an endothelin receptor antagonist, pirfenidone, nintedanib, compound embryonic bovine liver extract, asiaticoside, D-penicillamine, vitexin, 5-fluorouracil, or relaxin peptide analog B7-33. The macrophage colony-stimulating factor-1 receptor inhibitor includes at least one of GW2580, BLZ945, PLX3397, ARRY-382, PLX7486, or JNJ-40346527.

In some embodiments, the pro-angiogenic component includes at least one of vascular endothelial growth factor (VEGF), or platelet derived growth factor (PDGF).

In some embodiments, the anti-allergic component includes at least one of mometasone furoate, desonide, triamcinolone acetonide, fluticasone propionate, or halometasone.

In some embodiments, the functional component includes at least one of dexamethasone, a macrophage colony-stimulating factor-1 receptor inhibitor, enoxaparin sodium, interferon, VEGF, or mometasone furoate.

In some embodiments, the drug formulation 30 further includes at least one of an excipient or a pro-osmotic agent.

In some embodiments, the excipient includes at least one of collagen, bovine serum albumin, gelatin, chitosan, hyaluronic acid, chondroitin sulfate, sodium alginate, poly(vinyl alcohol), poly(ethylene glycol), poly(vinylpyrrolidone), poly(lactic-co-glycolic acid) copolymer, PLGA, triglycerides, dextrin, hydroxy sodium methylcellulose, petroleum jelly, propylene glycol, glycerin, stearic acid, glyceryl monostearate, lanolin, beeswax, paraffin wax, liquid paraffin wax, triethanolamine, Span-80 ( chemically a fatty acid ester of water-loss sorbitan), or an emulsifier OP (Emulsifier OP). The emulsifier OP may be Emulsifier OP-10 (Emulsifier OP-10, chemically an alkylphenol polyoxyethylene ether).

By adding an excipient, it is beneficial to prepare the drug formulations 30 containing different functional components into dosage forms with different morphologies, which in turn helps to set the drug formulations 30 containing different functional components at different locations of the analyte sensor 1 as needed, thereby achieving the purpose of delivering the drug formulations 30 containing different functional components to different target sites.

In some embodiments, the osmotic promoter includes at least one of lecithin, ethanol, propylene glycol, ethyl acetate, dimethyl sulfoxide, lauryl nitrone, oleic acid, lauryl alcohol, urea, salicylic acid, dimethyl amino acid ester, or limonene. By adding the osmotic promoter, it is beneficial to promote absorption of the drug formulation 30 into the skin, thereby allowing better functioning of the functional components of the drug formulation 30.

In some embodiments, a form of the drug formulation 30 includes at least one of a coating, a gel, an emulsion, a paste, or a microcapsule.

In some embodiments, as shown in FIG. 2, the drug formulation 30 is provided at an end of the accommodating groove of the auxiliary needle 20 proximate to the needle tip 21. In this way, when the auxiliary needle 20 is withdrawn, the drug formulation 30 is retained under the skin with the assistance of the sensing probe 10. In some embodiments, the drug formulation 30 may be in a gel form, or a microencapsulated form. In some embodiments, the functional component of the drug formulation 30 includes at least one of an anti-inflammatory component, an anti-coagulant component, or an anti-fibrotic component.

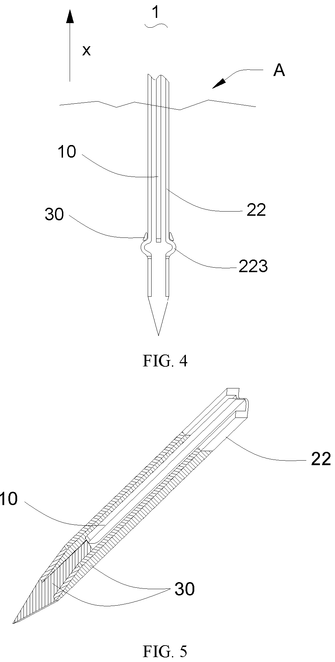

In some embodiments, as shown in FIG. 5, the drug formulation 30 may be disposed on an inner surface of the side wall 22 of the auxiliary needle 20, on an inner surface of the bottom wall 23 of the auxiliary needle 20, or on an outer surface of the side wall 22 of the auxiliary needle 20. In some embodiments, a form of the drug formulation 30 may be at least one of a coating, a gel, an emulsion, or a cream. The functional component of the drug formulation 30 includes at least one of an anti-inflammatory component or an anti-allergic component.

In some embodiments, as shown in FIG. 6, the drug formulation 30 may be disposed on the inner surface of the side wall 22 of the auxiliary needle 20; and/or, the drug formulation 30 is disposed on the inner surface of the bottom wall 23 of the auxiliary needle 20. In some embodiments, a form of the drug formulation 30 may be at least one of a coating, a gel, an emulsion, or a cream. The functional component of the drug formulation 30 includes at least one of an anti-inflammatory component or an anti-allergic component.

In some embodiments, as shown in FIG. 7, the probe substrate 11 further includes a second protrusion 113, and the drug formulation 30 is provided on the inner surface of the side wall 22 of the auxiliary needle 20; and/or, the drug formulation 30 is provided on the inner surface of the bottom wall 23 of the auxiliary needle 20. In this way, when the auxiliary needle 20 is withdrawn, the drug formulation 30 is retained subcutaneously with assistance of the second protrusion 113 on the probe substrate 11 under the action of the skin A. In some embodiments, a form of the drug formulation 30 may be one or more of a coating, a gel, an emulsion, or a cream. The functional component of the drug formulation 30 includes at least one of an anti-inflammatory component, an anti-coagulant component, an anti-fibrotic component, or a pro-angiogenic component. In some embodiments, the probe substrate 11 further includes two second protrusions 113.

In some embodiments, as shown in FIGs. 5 and 8, the drug formulation 30 is provided on the outer surface of the side wall 22 of the auxiliary needle 20. During inserting needle of the auxiliary needle 20, the drug formulation 30 is retained on the surface of the skin A due to the action of the skin A (i.e., the drug formulation 31 retained at the target site). In some embodiments, a form of the drug formulation 30 may be at least one of a coating, a gel, an emulsion, a paste, a microcapsule. Functional components of the drug formulation 30 include at least one of an anti-allergic component or an anti-inflammatory component.

In some embodiments, as shown in FIG. 3, the side wall 22 of the auxiliary needle 20 is further provided with a first protrusion 223, and the drug formulation 30 is provided at a position of the outer wall of the auxiliary needle 20 where the first protrusion 223 is away from the needle tip 21 of the analyte sensor 1. In this way, when the auxiliary needle 20 is inserted, the drug formulation 30 can be smoothly delivered under the skin due to protection of the first protrusion 223; when the auxiliary needle 20 is withdrawn, the drug formulation 30 is retained under the skin due to the skin A. When the auxiliary needle 20 is withdrawn, the drug formulation 30 is retained under the skin due to the skin A. In some embodiments, a form of the drug formulation 30 may be at least one of a coating, a gel, an emulsion, a cream, or a microencapsulation. The functional component of the drug formulation 30 includes at least one of an anti-inflammatory component, an anti-coagulant component, an anti-fibrotic component, or a pro-angiogenic component.

In some embodiments, referring to FIGs. 11-16, the drug formulation 30 is disposed on at least one of the surface of the working electrode 122, the surface of the reference electrode 123, the surface of the counter electrode 121, the first blank region 1121, the second blank region 1122, the third blank region 1123, the fourth blank region 1124, or the fifth blank region 1125 of the sensing probe 10.

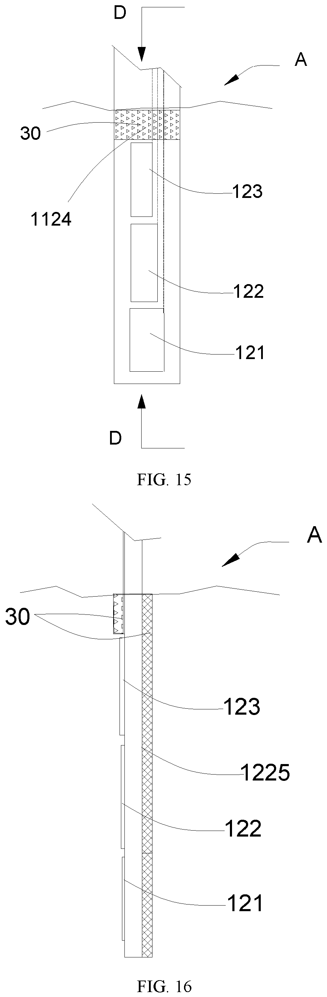

In some embodiments, referring to FIGs. 15 and 16, the drug formulation 30 is provided on at least one of the surface of the reference electrode 123, the surface of the counter electrode 121, the first blank region 1121, the second blank region 1122, the third blank region 1123, the fourth blank region 1124, or the fifth blank region 1125. By providing the drug formulation 30 on the electrode region 111 of the non-working electrode 122 of the sensing probe 10, on the one hand, it does not impair the function of the analyte sensor 1, and on the other hand, it provides a controlled local environment around the analyte sensor based on sustained release of the drug to achieve acute inflammation suppression and sustained local anti-inflammation to bypass the FBR and fibrosis, thereby improving function and service life of the analyte sensor 1 and comfort.

In some embodiments, a plurality of drug formulations 30 with different morphologies can be provided simultaneously at different locations of the analyte sensor 1. In some embodiments, the drug formulations 30 are provided on the outer surface of the side wall 22 of the auxiliary needle 20 and in the fifth blank region 1125. In this way, different drug formulations 30 can be delivered simultaneously and in a targeted manner to different target sites, thereby the drug formulations 30 containing different functional components can be delivered simultaneously, allowing better functioning of the functional components of the drug formulations 30.

In some embodiments, a sensor of the sensing probe 10 is selected from at least one of an electrochemical sensor or a biosensor. In some embodiments, the sensor of the sensing probe 10 is an electrochemical sensor including a current-type, voltage-type, capacitive-type, or impedance-type electrochemical sensor.

The present invention further provides a method for preparing the analyte sensor 1. The method includes: preparing a drug formulation 30; disposing the drug formulation 30 on the auxiliary needle 20 and/or the sensing probe 10; and, disposing the sensing probe 10 in an accommodating groove of the auxiliary needle 20.

In some embodiments, the drug formulation 30 is provided on the auxiliary needle 20 by at least one of methods including in-hole spot coating, surface spot coating, dip coating, spin coating, or spray coating. In some embodiments, the drug formulation 30 is provided on the sensing probe 10 by at least one of methods including in-hole dot coating, surface dot coating, dip coating, spin coating, or spray coating.

The present invention also provides a method of applying the analyte sensor 1, including: percutaneously piercing the analyte sensor 1 into a target site; and, withdrawing the auxiliary needle 20 and leaving the sensing probe 10 at the target site. In some embodiments, the target site is subcutaneous tissue.

Ingredients in the embodiments of the present invention are commercially available.

### Preparation of drug formulations

### Example 1

A solution of PVP (K-30, i.e., Polyvinylpyrrolidone K30) with a mass concentration of 0.5 wt% was prepared, and then a functional component was added, dispersed, and a functional component solution was prepared. Among them, a mass concentration of the functional component was controlled to be 0.1 wt% of a coating after drying (i.e., a mass ratio of PVP (K-30) to the functional component was 1000 : 1). The functional component solution was sprayed on a surface of the sensory probe under conditions of ultrasonic sprayer with an ultrasonic frequency of 120 kHz, ultrasonic nozzle with a power of 1.5 W, nozzle moving speed of 20 mm/s, and carrier gas pressure of 6 kPa. The number of spraying passes was 10, thereby obtaining a sensing probe including the functional component coating.

In some embodiments, the functional component may be at least one of an anti-inflammatory component, an anti-coagulant component, an anti-fibrotic component, or a pro-angiogenic drug component.

### Example 2

0.6 g of sodium hydroxymethylcellulose was dissolved with 10.0 g of deionized water by stirring, and then 1.5 g of glycerol was added and stirred well to obtain a hydrogel matrix. A functional component was added to the hydrogel matrix to obtain a drug formulation 30 in a hydrogel form.

In some embodiments, the functional component may be at least one of an anti-inflammatory component, an anti-coagulant component, an anti-fibrosis component, a pro-angiogenic component,or an anti-allergic component.

### Example 3

1.2 g of stearic acid, 0.35 g of glycerol ester of monostearic acid, 0.6 g of liquid paraffin, 0.1 g of petroleum jelly, and 0.5 g of lanolin were melted in a water bath with heat, and the temperature was maintained at 80° C. After complete melting, 0.04 g of triethanolamine and 10.0 g of deionized water preheated at 80° C were added and continuously stirred clockwise while adding, until it solidified in a form of a milky-white semi-solid, and then it was solidified by stirring at room temperature until near condensation to obtain an emulsion matrix. A functional component was added to the emulsion matrix to obtain a drug formulation 30 in an emulsion form.

In some embodiments, the functional component may be at least one of an anti-inflammatory component or an anti-allergic component.

### Example 4

4.0 g of glyceryl monostearate and 4.0 g of paraffin were melted in a water bath with heating, then 2.0 g of petroleum jelly, 20.0 g of liquid paraffin and 0.1 g of Span-80 were added while the temperature was maintained at 80° C. After complete melting, 0.2 g of OP emulsifier preheated at 80° C and 10.0 g of deionized water were added, and the mixture was stirred continuously clockwise while adding until a milky-white semi-solid was solidified, and a paste matrix was obtained. A functional component was added to the paste matrix to obtain a drug formulation 30 in a paste form.

In some embodiments, the functional component may be at least one of an anti-inflammatory component or an anti-allergic component.

### Example 5

40 g/L of a sodium alginate solution was prepared using sodium alginate with a molecular weight of 80 k - 120 k and sterilized at 121 ° C for 15 min. 4 g/L of a solution of low molecular weight chitosan was prepared using low molecular weight chitosan (molecular weight typically <10k, ≥ 75% deacetylation) and 90 mL of deionized water. The solution of low molecular weight chitosan was acidified by adding 0.4 mL of glacial acetic acid, and adjusted pH to 5.7-6.0 by adding 1 M of NaOH solution. A volume of the solution of low molecular weight chitosan was adjusted to 100 mL by adding deionized water after filtration and then sterilized at 121°C for 15 min to obtain a chitosan solution. A functional component was dispersed in the sodium alginate solution at 1 wt% to prepare a dispersion of functional component. 0.1 M of CaCl₂ sterile solution was used as a hardening solution and the dispersion of functional component were dripped in the hardening solution through a needle with a size of 0.11 mm, and the dispersion of functional component was gelated for 30 min and then cleaned with deionized water. Cleaned gel beads were then added to 100 mL of chitosan solution by cyclotron shaking at 100 rpm and then washed with deionized water to obtain chitosan-coated microcapsules.

In some embodiments, the functional component is at least one of an anti-inflammatory component, an anti-coagulant component, an anti-fibrotic component, a pro-angiogenic component, or an anti-allergic component.

### Example 6

Appropriate amounts of a functional component and 50 mg of bovine serum albumin (Sigma, SRE0098) were dissolved in 1 mL of deionized water as an internal aqueous phase. PLGA (poly(lactic-co-glycolic acid) was dissolved in dichloromethane to prepare 20 mL of PLGA solution at a concentration of 50 g/L as an oil phase. Polyvinyl alcohol was dissolved in deionized water to prepare an aqueous solution of polyvinyl alcohol with a mass concentration of 10 g/L as an external aqueous phase. 100 µL of the inner aqueous phase was added to 2 mL of the oil phase, and a primary emulsion was formed by oscillating with an ultrasonic breaker probe at 60% amplitude (ultrasonic power of 75 W) for 30 s in an ice bath. The primary emulsion was then added to 20 mL of the external aqueous phase, and a dispersion obtained was immediately spray-dried with a mini-spray dryer at a flow rate of 1 mL/min and an inlet/outlet temperature of 25°C/23°C. Spray-dried particles were washed out from a cyclone of a spray dryer with 0.05% Poloxamer 188, collected on acetate membrane filters, then vacuum dried at room temperature and finally stored at -20°C under dry conditions.

In some embodiments, the functional component is at least one of an anti-inflammatory component, an anti-coagulant component, an anti-fibrotic component, a pro-angiogenic component, or an anti-allergic component.

### Method of applying a drug formulation

### Application example 1

Polyvinyl alcohol gels loaded with dexamethasone were prepared by physical cross-linking of polyvinyl alcohol using a "freeze-thaw" method. Firstly, polyvinyl alcohol (molecular weight: ~10k) was dissolved by stirring under heating at 85°C to obtain 15 wt% polyvinyl alcohol solution, then dexamethasone was added to form a dexamethasone solution with a dexamethasone content of 0.1 wt%, and stirring was continued for 2 h. After ultra-sonication for 30 min to remove air bubbles, the product was put into the freezing zone at -20°C for 1 h, and then thawed at room temperature (about 25°C) for 1 h. Such freezing-thawing procedure was carried out for three times. The polyvinyl alcohol gels loaded with dexamethasone were obtained by the "freeze-thaw" method.

As shown in FIG. 2, a volume of the drug-loaded polyvinyl alcohol gel was dispensed onto a distal end of the accommodating groove of the auxiliary needle 20 of the analyte sensor 1. After implantation of the sensing probe 10 and withdrawal of the auxiliary needle 20, the drug-loaded polyvinyl alcohol gel at the distal end of the sensing probe 10 was retained at an implantation site for sustained release of dexamethasone, which provides an anti-inflammatory effect to improve functionality and a service life of the analyte sensor 1.

### Application example 2

PLGA microspheres for VEGF were prepared by the method of Example 5. Hyaluronic acid and polyethylene glycol were dissolved at a content of 8 wt% and 2 wt%, respectively. The PLGA microspheres loaded VEGF were thoroughly mixed with the hyaluronic acid/polyethylene glycol hydrogel at a weight ratio of VEGF at 0.2 wt% to obtain a drug-loaded hyaluronic acid/polyethylene glycol hydrogel.

As shown in FIGs. 3 and 4, a certain volume of drug-loaded hyaluronic acid/polyethylene glycol hydrogel was dispensed onto a location of a first protrusion 223 of the auxiliary needle 20 of the analyte sensor 1 away from a distal end of the auxiliary needle 20. After implantation of the sensing probe 10 and withdrawal of the auxiliary needle 20, the drug-loaded hyaluronic acid/polyethylene glycol hydrogel was retained at an implantation site using the subcutaneous tissue at the implantation site to continuously release VEGF, which provides a pro-angiogenic effect to improve functionality and a service life of the analyte sensor 1.

### Application example 3

An emulsion matrix was prepared by the method of Example 3, and then mometasone furoate (0.2 wt%) was added to obtain a mometasone furoate emulsion. The mometasone furoate emulsion was coated on an outer surface of the auxiliary needle 20.

As shown in FIG. 8, when the sensing probe 10 was implanted, the drug-loaded emulsion was retained at a supradermal area of an implantation site to continuously release mometasone furoate to provide an anti-allergic effect to improve comfortability of the analyte sensor 1 when using.

### Application example 4

Microcapsules were prepared by encapsulating interferon with sodium alginate as a wall material according to the method of Example 5. 2 wt% chitosan solution and 2 wt% acid-soluble collagen solution were prepared using 0.1 M and 0.02 M aqueous acetic acid as solvents, respectively, and 50 wt% sodium glycerophosphate solution was prepared using deionized water as solvent. The above three solutions were then stirred at 4 °C for 1 h. The chitosan solution and acid-soluble collagen solution were mixed in a ratio of 1:1 at a temperature of 4 °C, and then sodium glycerophosphate solution was added dropwise to make a final concentration of sodium glycerophosphate solution to be 6 wt%, and a synthesized sol was stored at 4 °C. At a temperature of 4°C, the interferon microcapsules and GW2580 were thoroughly mixed with the synthesized sol, and the sensing probe 10 was immersed in an obtained drug-loaded sol, and then the temperature was raised to 37°C.

As shown in FIGs. 11 and 12, a gel coating loaded with interferon and GW2580 was obtained. After implantation of the sensing probe 10, the drug-loaded gel coating was retained at an implantation site together with the sensing probe 10 to continuously release the interferon and GW2580, which provides anti-inflammatory and anti-fibrotic effects, and thus improves functionality and a service life of the analyte sensor 1.

### Application example 5

Gelatin aqueous solution (1 wt%) was dissolved by heating at 50° C and stirring sufficiently, and then curcumin (0.05 wt%) and prednisone (0.15 wt%) were added in accordance with a proportion of the drug to the coating. After mixing sufficiently, an obtained mixture was dot-coated on a first blank region 1121 and a second blank region 1122 of a surface of the sensing probe 10, and then dried to obtain a drug-loaded gelatin coating.

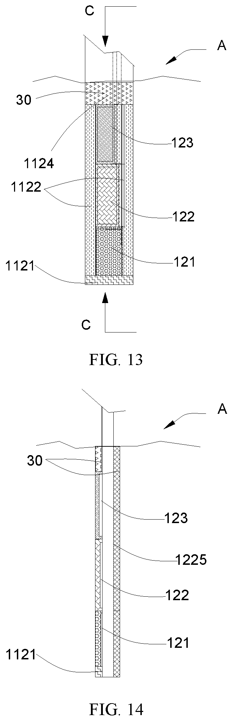

As shown in FIGs. 13 and 14, when the sensing probe 10 was implanted, the drug-loaded gelatin coating continuously released curcumin and prednisone at an implantation site, which provides anti-inflammatory and anti-fibrotic effects to improve functionality and a service life of the analyte sensor 1.

### Application example 6

A paste matrix was prepared according to the method of Example 4, and then desonide (0.3 wt%) was added to obtain a desonide paste. The desonide paste was coated on an outer surface of the side wall 22 of the auxiliary needle 20.

Polyethylene glycol molecular monomer (molecular weight was 20k) and N,N-methylenebisacrylamide (as a cross-linking agent) were dissolved in deionized water, and ammonium persulfate was added under nitrogen protection to obtain a 1 wt% aqueous ammonium persulfate solution, after which the reaction was stirred at 50°C for 3 h. The product was extracted with water. Then the product was stirred and mixed with 0.1 wt% dexamethasone and 0.5 wt% enoxaparin sodium to obtain dexamethasone sol and enoxaparin sodium sol, respectively. The dexamethasone sol was spot-coated on a fourth blank region 1124 of the analyte sensor 1, and the enoxaparin sodium sol was sprayed on a fifth blank region 1125 of the analyte sensor 1. After drying, the dexamethasone gel coating was obtained on a neck portion of the sensing probe, and the enoxaparin sodium gel coating was obtained on a back side of the sensing probe relative to a side of the sensing probe having the spot electrode 12.

As shown in FIGs. 6, 15 and 16, during implantation of the sensing probe 10, the desonide paste was retained on a supradermal part of an implantation site for continuous release of the dexedrine, and the dexamethasone coating and the enoxaparin sodium coating for continuous release of the dexamethasone and the sodium heparin at the implantation site provided an antiallergenic, anti-inflammatory, and anti-coagulant effects, improving functionality and a service life of the analyte sensor 1.

The technical features of the above-mentioned embodiments can be combined arbitrarily. In order to make the description concise, not all possible combinations of the technical features are described in the embodiments. However, as long as there is no contradiction in the combination of these technical features, the combinations should be considered as in the scope of the present invention.

The above-described embodiments express only several embodiments of the present invention, which are described in a more specific and detailed manner, but are not to be construed as a limitation of the scope of the patent application. It should be pointed out that for a person of ordinary skill in the art, several deformations and improvements can be made without departing from the conception of the present invention, which all fall within the scope of protection of the present invention. Therefore, the scope of protection of the present invention shall be subject to the attached claims.

## Claims

1. An analyte sensor (1), **characterized by** comprising a sensing probe (10), an auxiliary needle (20) and a drug formulation (30),
wherein the auxiliary needle (20) comprises a needle tip (21), a side wall (22) and a bottom wall (23), the needle tip (21) is connected to the bottom wall (23), an accommodating groove (24) is defined by the side wall (22) and the bottom wall (23) connected to each other, the accommodating groove (24) is configured to accommodate the sensing probe (10); and
the drug formulation (30) is disposed on the auxiliary needle (20) and/or on the sensing probe (10).

2. The analyte sensor (1) of claim 1, wherein the side wall (22) of the auxiliary needle (20) comprises a first portion (221), the first portion (221) of the side wall (22) is located at an end of the side wall (22) proximate to the needle tip (21), and a height of the first portion (221) perpendicular to the bottom wall (23) decreases in a direction proximate to the needle tip (21).

3. The analyte sensor (1) of claims 1 or 2, wherein a first protrusion (223) is further provided on an outer surface of the side wall (22) of the auxiliary needle (20).

4. The analyte sensor (1) of any one of claims 1 to 3, wherein the sensing probe (10) comprises a probe substrate (11) and an electrode (12), the probe substrate (11) comprises an electrode region (111) and a blank region (112), the electrode (12) is disposed on the electrode region (111) of the probe substrate (11).

5. The analyte sensor (1) of claim 4, wherein the blank region (112) comprises a first blank region (1121), a second blank region (1122), a third blank region (1123), a fourth blank region (1124) and a fifth blank region (1125),
the first blank region (1121) is located at one end of the probe substrate (11) adjacent to the needle tip (21) of the auxiliary needle (20), the second blank region (1122) is located on both sides of the electrode region (111) of the probe substrate (11), the third blank region (1123) is located on both sides of the probe substrate (11), the fourth blank region (1124) is located at one end of the probe substrate (11) away from the needle tip (21) of the auxiliary needle (20), and the fifth blank region (1125) is located on an opposite side of the probe substrate (11) relative to a side of the probe substrate (11) having the electrode (12).

6. The analyte sensor (1) of any one of claims 1 to 5, wherein the sensing probe (10) comprises a probe substrate (11), the probe substrate (11) further comprises a second protrusion (113), and the second protrusion (113) is disposed between the probe substrate (11) and the side wall (22) of the auxiliary needle (20).

7. The analyte sensor (1) of any one of claims 1 to 6, wherein the drug formulation (30) comprises a functional component, the functional component comprises at least one of an anti-inflammatory component, an anti-coagulant component, an anti-fibrotic component, a pro-angiogenic component, or an anti-allergic component.

8. The analyte sensor (1) of claim 7, wherein the anti-inflammatory component comprises at least one of curcumin, dexamethasone, prednisone, methyl-prednisone, betamethasone, beclomethasone propionate, prednisolone, hydrocortisone, or methylprednisolone;
the anti-coagulant component comprises at least of sodium heparin, calcium heparin, enoxaparin sodium, dalteparin sodium, nalteparin calcium, argatroban, bivalirudin, warfarin, dabigatran etexilate, rivaroxaban, apixaban, dipyridamole, aspirin, clopidogrel hydrogensulphate, urokinase, or antithrombin;
the anti-fibrotic component comprises at least one of a macrophage colony-stimulating factor-1 receptor inhibitor, colchicine, interferon, an endothelin receptor antagonist, pirfenidone, nintedanib, compound embryonic bovine liver extract, asiaticoside, D-penicillamine, retinoic acid, 5-fluorouracil, and relaxin peptide analogs B7-33;
the pro-angiogenic component comprises at least one of vascular endothelial growth factor, or platelet-derived growth factor; and
the anti-allergic component comprises at least one of mometasone furoate, desonide, triamcinolone acetonide, fluticasone propionate, or halometasone.

9. The analyte sensor (1) of claim 8, wherein the drug formulation (30) further comprises an excipient, a pro-osmotic agent, or any combination thereof.

10. The analyte sensor (1) of claim 9, wherein the excipient comprises at least one of collagen, bovine serum albumin, gelatin, chitosan, hyaluronic acid, chondroitin sulfate, sodium alginate, polyvinyl alcohol, polyethylene glycol, polyvinylpyrrolidone, poly(lactic acid-hydroxyacetic acid) copolymer, triglyceride, dextrin, sodium hydroxymethylcellulose, petroleum jelly, propylene glycol, glycerol, stearic acid, mono glyceryl stearate, lanolin, beeswax, paraffin, liquid paraffin, triethanolamine, Span-80, or emulsifier OP-10; and/or
the pro-osmotic agent comprises at least one of lecithin, ethanol, propylene glycol, ethyl acetate, dimethyl sulfoxide, lauric nitrone, oleic acid, lauryl alcohol, urea, salicylic acid, dimethyl amino acid ester, limonene.

11. The analyte sensor (1) of any one of claims 1 to 10, wherein a form of the drug formulation (30) comprises at least one of a coating, a gel, an emulsion, a paste, or a microcapsule.

12. The analyte sensor (1) of any one of claims 1 to 11, wherein the drug formulation (30) is disposed at an end of the accommodating groove (24) of the auxiliary needle (20) proximate to the needle tip (21).

13. The analyte sensor (1) of claim 12, wherein the drug formulation (30) comprises at least one of a functional component, and the functional component comprises an anti-inflammatory component, an anti-coagulant component, or an anti-fibrosis component.

14. The analyte sensor (1) of any one of claims 1 to 11, wherein the drug formulation (30) is disposed on an inner surface of the side wall (22) of the auxiliary needle (20); and/or, the drug formulation (30) is disposed on an inner surface of the bottom wall (23) of the auxiliary needle (20).

15. The analyte sensor (1) of claim 14, wherein the drug formulation (30) comprises a functional component, the functional component comprises an anti-inflammatory component, an anti-allergic component, or any combination thereof.

16. The analyte sensor (1) of any one of claims 1 to 11, wherein the drug formulation (30) is disposed on an outer surface of the side wall (22) of the auxiliary needle (20).

17. The analyte sensor (1) of claim 16, wherein the drug formulation (30) comprises a functional component, the functional component comprises an anti-inflammatory component, an anti-allergic component, or any combination thereof.

18. The analyte sensor (1) of claim 3, wherein the drug formulation (30) is disposed at a position of the first protrusion (223) away from the needle tip (21).

19. The analyte sensor (1) of claim 18, wherein the drug formulation (30) comprises a functional component, and the functional component comprises an anti-inflammatory component, an anti-coagulant component, an anti-fibrotic component, a pro-angiogenic component, or any combination thereof.

20. The analyte sensor (1) of claim 5, wherein the electrode (12) comprises a working electrode (122), a reference electrode (123), and a counter electrode (121), the drug formulation (30) is disposed on at least one of a surface of the working electrode (122), a surface of the reference electrode (123), a surface of the counter electrode (121), the first blank region (1121), the second blank region (1122), the third blank region (1123), the fourth blank region (1124), or the fifth blank region (1125).

21. The analyte sensor (1) of claim 16, wherein the drug formulation (30) is disposed on at least one of a surface of the reference electrode (123), a surface of the counter electrode (121), the first blank region (1121), the second blank region (1122), the third blank region (1123), the fourth blank region (1124), or the fifth blank region (1125).

22. The analyte sensor (1) of claim 5, wherein the drug formulation (30) is disposed on an outer surface of the side wall (22) of the auxiliary needle (20) and the fifth blank region (1125).

23. The analyte sensor (1) of claim 22, wherein the drug formulation (30) comprises a functional component, and the functional component comprises an anti-inflammatory component, an anti-coagulant component, or any combination thereof.

24. A method for preparing the analyte sensor (1) of any one of claims 1 to 23, **characterized by** comprising:
preparing the drug formulation (30);
disposing the drug formulation (30) on the auxiliary needle (20) and/or on the sensing probe (10); and
disposing the sensing probe (10) in the accommodating groove (24) of the auxiliary needle (20),
wherein the drug formulation (30) is disposed on the auxiliary needle (20) by a method of in-hole spot coating, surface spot coating, dip coating, spin coating, spray coating, or combination thereof; and/or
the drug formulation (30) is disposed on the sensing probe (10) by a method of in-hole spot coating, surface spot coating, dip coating, spin coating, spray coating, or combination thereof.

25. A method of applying the analyte sensor (1) of any one of claims 1 to 23 or an analyte sensor (1) prepared by the method of claim 24, **characterized by** comprising following steps:
percutaneously piercing the analyte sensor (1) into a target site; and
withdrawing the auxiliary needle (20) and leaving the sensing probe (10) at the target site.
